# EUROPEAN PATENT APPLICATION

(11) **EP 4 714 406 A1**
(43) Date of publication of application: **25.03.2026**
(21) Application number: 25203429.3
(22) Date of filing: 19.09.2025
(51) Int. Cl.: A61F 6/08, A61F 6/12

(54) **GELLHORN PESSARY WITH REMOVAL FEATURE**

(30) Priority: 19.09.2024 AU 2024219861
(71) Applicant: Sayco Pty Ltd, Moorabbin, Victoria 3189 (AU)
(72) Inventor: YOUNG, Tim, Moorabbin, 3189 (AU)
(74) Representative: Kransell & Wennborg KB

(57) **Abstract**

The invention provides a Gellhorn pessary (10) including a body (11) comprising a base (12) and a stem (13) extending therefrom. A finger-pull (14) is associated with the body and is adapted to receive at least one finger of a user to facilitate removal of the Gellhorn pessary. The finger-pull is moveable between an open orientation that facilitates removal of the Gellhorn pessary and a retained orientation in which it is retained relative to the stem of the Gellhorn pessary.

## Description

### FIELD OF INVENTION

The present invention relates to a Gellhorn pessary with a removal feature that facilitates removal of the pessary from a user. More particularly, the invention relates to a Gellhorn pessary that includes a finger-pull. The finger-pull is moveable between an open orientation that facilitates easy removal of the pessary and a retained orientation in which it is retained relative to the stem of the pessary.

### BACKGROUND ART

Pessaries are implantable devices that have been used for many decades for the treatment of Pelvic Organ Prolapse and Stress Urinary Incontinence. Pessaries are mainly categorized into two types, supporting pessaries and space-occupying pessaries. Support pessaries function by supporting the prolapse and space-occupying pessaries by filling the vaginal space. There are also lever type pessaries. For example, pessaries may include at least the following types: ring, ring with support, shelf, Shaatz, Gellhorn, Risser, Smith, cube, tandem cube, cup, oval, Hodge, Hodge with knob, Hodge with support, Gehrung, incontinence dish with support, donut, incontinence ring, incontinence dish, and Inflatoball and Inflatable Donut.

The most common types of pessaries are the ring pessary, available with and without support, and the Gellhorn pessary, available with short and long stems. Notably, ring pessaries are normally used for minor prolapses and Gellhorn pessaries are used for moderate to severe prolapses.

The ring pessary in its simplest form is a circular ring of inert material, such as PVC or latex, or silicone. However, ring pessaries often include a supporting membrane that extends between the inner edges of the ring. The supporting membrane generally includes a web of material extending within the boundary of the ring and having drain holes in the supporting membrane. Ring pessaries can generally be easily folded in half for insertion and may be removed by a user by hooking a finger under or over the rim of the ring pessary, tilting the ring pessary slightly, and gently pulling down and out of the vagina. However, this is not possible with a ring with a supporting membrane.

The Gellhorn pessary is a widely used pessary. The Gellhorn pessary has a circular base normally made out of silicone, about 3-5 mm thick. The base is slightly concave as this form assists in creating suction and keeping the Gellhorn in place. Similar to the ring pessary with support, the Gellhorn has drain holes in the base. The Gellhorn pessary is distinctive in that it includes a central stem located on the base of the Gellhorn pessary, the stem including a spherical knob at the distal end thereof. The central stem has a lumen or central drainage hole through it to assist in ensuring there is no build-up of exudate in the central base of the stem. The stem has an amount of firmness that assists in keeping the correct orientation of the Gellhorn pessary in use, and prevents it from flipping around whereby the stem would be incorrectly pointing upwards. The conventional straight, cylindrical form of the stem does not allow a clinician or user to get sufficient purchase or grip on the stem knob. A recent attempt to rectify this provided a pessary with a tapered stem and a mushroom shaped head allowing for a better grip. However, the tapered nature of this stem disadvantageously made it easily collapsible as the wall of the stem becomes very narrow at the uppermost part of the stem due to the presence of the central drainage hole.

To insert the Gellhorn pessary the stem, which is flexible, is bent or compressed to the base and the base is inserted in-line with the vaginal opening. Removal of the Gellhorn pessary is generally difficult due to the suction created by the concave circular base of the pessary. Clinicians often need to use a device such as sponge forceps to grip the stem, placing a finger up and around the base to release the suction, and move the base downwards to remove the pessary. As such, in its current form the Gellhorn pessary is difficult to remove by a clinician and is generally considered to be a pessary that a patient cannot remove by themselves.

Another widely used space-occupying pessary is the cube pessary. This type of pessary includes a cube body, generally including drain holes, and a cord for grasping in order to remove the pessary. The cord is generally attached to the cube body with an anchor. The cord may also be looped with two anchors securing each end of the loop to the cube body through the drain holes. However, such cord and anchor systems have the disadvantage of potential breakage to leave material inside the user after removal of the cube pessary and making the pessary difficult to remove for the user. In addition, pulling on the cord without deforming the walls of the cube pessary (thereby breaking the suction) may cause the pessary to pull the prolapse down as it is removed.

Generally, these types of pessaries have been used in their current forms for a number of decades. More particularly, space-filling pessaries such as the cube and Gellhorn pessaries have been used in their present form with little consideration given to facilitating ease of removal by a user.

The subject matter claimed herein is not limited to embodiments that solve any disadvantages or that operate only in environments such as those described above. Rather, this background is only provided to illustrate exemplary technology areas where some embodiments described herein may be practiced.

Various aspects and embodiments of the invention will now be described.

### SUMMARY OF INVENTION

The present invention relates to a Gellhorn pessary with a removal feature that facilitates removal of the pessary from a user. More particularly, the invention relates to a Gellhorn pessary that includes a finger-pull. The finger-pull is moveable between an open orientation that facilitates easy removal of the pessary and a retained orientation in which it is retained relative to the stem of the pessary.

According to one aspect of the invention there is provided a Gellhorn pessary comprising:
a body comprising a base and a stem extending therefrom; and
a finger-pull associated with the body portion and adapted to receive at least one finger of a user to facilitate removal of the Gellhorn pessary,
wherein the finger-pull is moveable between an open orientation that facilitates removal of the Gellhorn pessary and a retained orientation in which it is retained relative to the stem of the Gellhorn pessary.

It is considered that having the finger-pull moveable between the open orientation and the retained orientation will provide a user with easy access to the finger-pull for removal of the Gellhorn pessary when in the open orientation, while minimising discomfort that may be caused by the finger-pull during use in the retained orientation. That is, the finger-pull preferably does not significantly outwardly extend from the base of the Gellhorn pessary in the retained orientation.

The finger-pull preferably comprises a substantially V-shaped supporting web hingedly connected with the base of the Gellhorn pessary and a loop extending from the V-shaped supporting web. The supporting web advantageously minimises deformation of the finger-pull and transfers the force applied to the body of the Gellhorn pessary. This further minimises the force required to break suction between the body of the Gellhorn pessary and the vaginal wall. The application of user pulling force on the loop deforms the body thereby releasing suction between outer edges of the body of the Gellhorn pessary and vaginal cavity surfaces they are contacting, in use.

It is envisaged that the finger-pull may be retained relative to the stem of the Gellhorn pessary by any suitable means. In a preferred embodiment, the stem of the Gellhorn pessary comprises a solid tapered shaft and a distal knob. The distal knob preferably comprises a tab on an underside thereof, the tab adapted to receive a distal portion of the loop and thereby retain the finger-pull relative to the stem in the retained orientation. To assist in the retention under the tab of the distal knob, the distal end of the loop preferably has a concave form. The concave form of the loop results in the loop effectively bending around the stem, which may minimise potential contact with the user's mucous membranes.

To facilitate close securing of the finger-pull relative to the base of the Gellhorn pessary, the base preferably comprises a recessed portion on an upper surface thereof, the recessed portion adapted to receive the V-shaped supporting web when the finger-pull is in the retained orientation. The recessed portion preferably comprises at least one tab, preferably two opposing tabs, adapted to retain the V-shaped supporting web in the recessed portion, the tabs of the recessed portion preferably extending from lateral sides of the recessed portion.

Preferably, the finger-pull is a unitary structure formed from a resilient polymeric material, the loop being flexible between a planar orientation relative to the V-shaped supporting web, in the open orientation, and a perpendicular orientation relative to the V-shaped supporting web, in the retained orientation. This may advantageously provide added comfort to a user of the Gellhorn pessary as, in the retained orientation, the loop extends perpendicularly from the base of the Gellhorn pessary and close to the stem.

The base of the Gellhorn pessary preferably comprises a plurality of drainage apertures. The drainage apertures are preferably disposed with generally even spacing around the base of the Gellhorn pessary. Preferably, the stem of the body comprises a drainage port extending from an underside of the base, at an angle through the base of the Gellhorn pessary, to an opening preferably at a base of the stem. This central angled port may be easily formed as a part of an injection moulding process and obviates the need for a central hole through the stem part of the Gellhorn. The lack of a central stem hole advantageously allows for a tapered stem with a mushroom style head for grip assistance, but with enough stem firmness to ensure the stem does not collapse easily.

The finger-pull preferably comprises a plurality of drainage outlets. More particularly, the finger-pull preferably comprises drainage outlets formed as apertures, for example circular apertures, and/or a longitudinal slit, advantageously ensuring no fluid build-up is possible above the V-shaped supporting web.

The body of the Gellhorn pessary is preferably a unitary piece of material, preferably formed from silicone. The finger-pull is also preferably formed from silicone. The Gellhorn pessary may be a unitary device formed from a one-mould process.

### BRIEF DESCRIPTION OF ACCOMPANYING DRAWINGS

To further clarify various aspects of some embodiments of the present invention, a more particular description of the invention will be rendered by references to specific embodiments thereof, which are illustrated in the appended drawings. It should be appreciated that these drawings depict only typical embodiments of the invention and are therefore not to be considered limiting on its scope. The invention will be described and explained with additional specificity and detail through the accompanying drawings in which:
FIG. 1 illustrates a perspective view of a Gellhorn pessary accordingly to an embodiment of the invention with the finger-pull in a retained orientation.
FIG. 2 illustrates a rear view of the Gellhorn pessary of Figure 1 with the finger-pull in an open orientation.
FIG. 3 illustrates a plan view of the Gellhorn pessary of Figure 1.
FIG. 4 illustrates a bottom view of the Gellhorn pessary of Figure 1.
FIG 5 illustrates a front view of the Gellhorn pessary of Figure 1.
FIG. 6 illustrates a side view of the Gellhorn pessary of Figure 1.
FIG. 7 illustrates a rear view of the Gellhorn pessary of Figure 1.
FIG. 8 illustrates a plan view of a Gellhorn pessary with the finger-pull in an open orientation.
FIG. 9 illustrates a rear view of the Gellhorn pessary of Figure 8.
FIG. 10 illustrates a sectional side view of the Gellhorn pessary of Figure 8.

### DETAILED DESCRIPTION OF THE PREFERRED EMBODIMENTS

Hereinafter, this specification will describe the present invention according to the preferred embodiments. It is to be understood that limiting the description to the preferred embodiments of the invention is merely to facilitate discussion of the present invention and it is envisioned without departing from the scope of the appended claims.

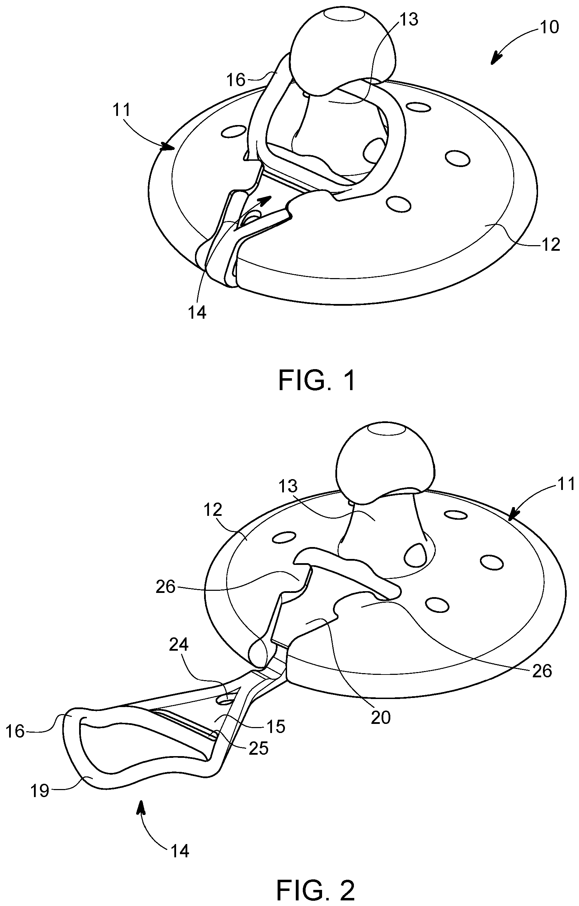

Referring to Figures 1 to 7, a Gellhorn pessary 10 is illustrated. The Gellhorn pessary 10 includes a body 11 comprising a base 12 and a stem 13 extending therefrom. A finger-pull 14 is associated with the body 11 and is adapted to receive at least one finger of a user to facilitate removal of the Gellhorn pessary 10. The finger-pull 14 is moveable between an open orientation, illustrated in Figure 2, that facilitates removal of the Gellhorn pessary 10 and a retained orientation, illustrated in Figure 1, in which it is retained relative to the stem 13 of the Gellhorn pessary 10.

When the finger-pull 14 is in the open orientation it extends outwardly from the body 11 of the Gellhorn pessary 10, providing a user with easy access to the finger-pull 14 for removal of the Gellhorn pessary 10. When in the retained orientation, the position of the finger-pull 14 may advantageously minimise discomfort that may be caused by the finger-pull 14 during use in the retained orientation.

The finger-pull 14 comprises a substantially V-shaped supporting web 15 hingedly connected with the base 12 of the Gellhorn pessary 10 and a loop 16 extending from the V-shaped supporting web 15. As previously noted, the V-shaped supporting web 15 advantageously minimises deformation of the finger-pull 14 and transfers the force applied to the body 11 of the Gellhorn pessary 10.

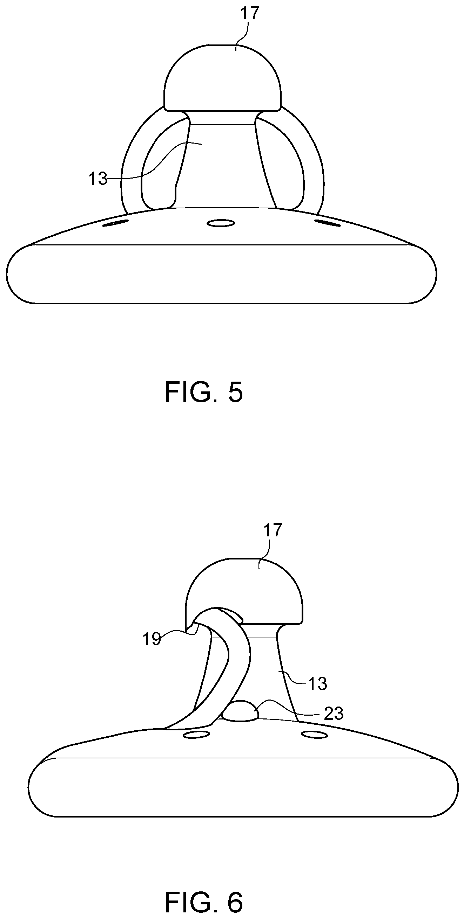

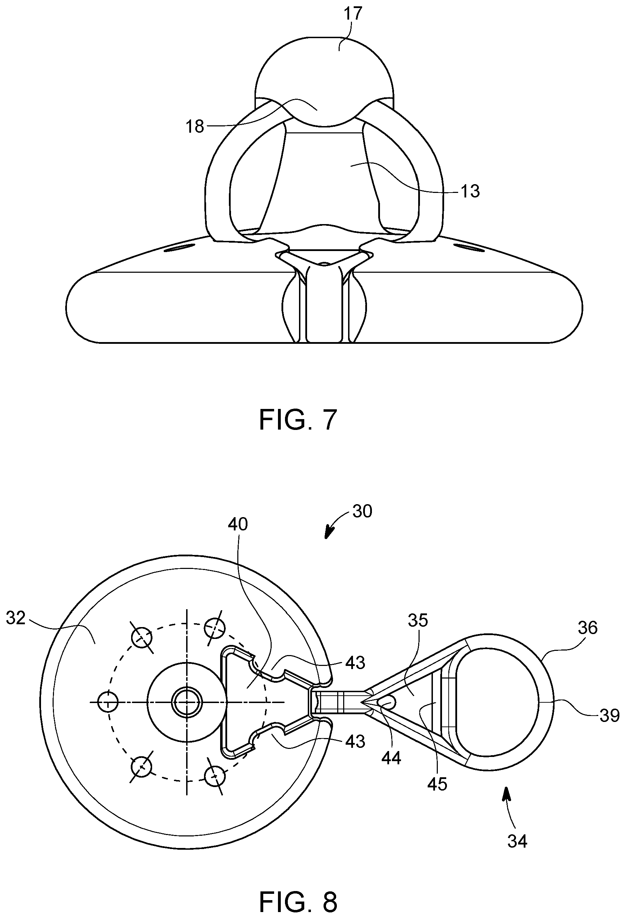

As best seen in Figures 6 and 7, the stem 13 of the Gellhorn pessary 10 comprises a distal knob 17 that includes a tab 18 on an underside thereof. The tab 18 is adapted to receive a distal portion 19 of the loop 16 and thereby retain the finger-pull 14 relative to the stem 13 in the retained orientation. The tab 18 extends further down than the rest of the distal knob 17 to retain the finger-pull 14 as it is clipped in.

The base 11 comprises a recessed portion 20, best seen in Figure 2, on an upper surface thereof. The recessed portion 20 receives the V-shaped supporting web 15 when the finger-pull 14 is in the retained orientation, as illustrated in Figure 1. The recessed portion 20 comprises two opposing tabs 26 extending from lateral sides of the recessed portion 20 and adapted to retain the V-shaped supporting web 15 in the recessed portion 20.

The finger-pull 14 is a unitary structure formed from a resilient polymeric material, the loop 16 being flexible between a planar orientation relative to the V-shaped supporting web 15 as shown in Figure 2, and a perpendicular orientation relative to the V-shaped supporting web 15 as shown in Figure 1. In the retained orientation the loop 16 extends perpendicularly from the base 12 of the Gellhorn pessary 10 and close to the stem 13, and forms a curve around the stem, minimising its footprint and providing added comfort to the user.

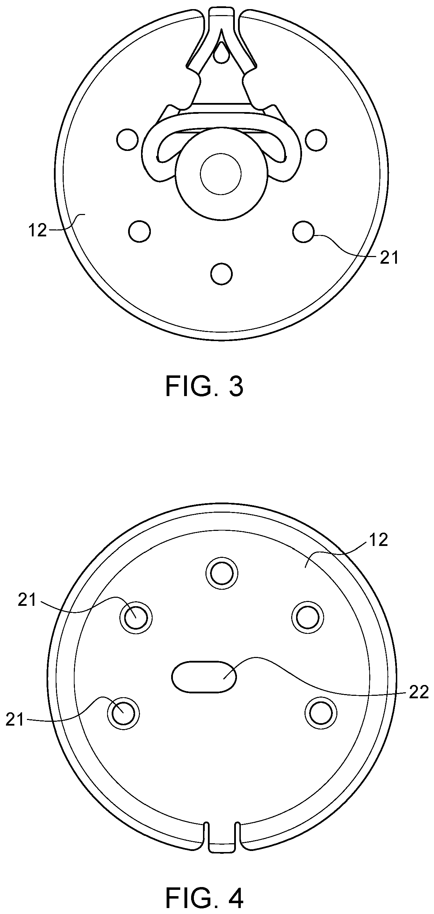

As best illustrated in Figures 3 and 4, the base 12 of the Gellhorn pessary 10 comprises a plurality of drainage apertures 21. The drainage apertures 21 are disposed evenly around the base 12 of the Gellhorn pessary 10 and allow fluid to drain through the Gellhorn pessary 10 in use. The base 12 of the body 11 also comprises a drainage port 22 extending from an underside of the base 12, at an angle through the base 12, to an opening 23 (see Figure 6) at the base of the stem 13. This forms a drainage ramp for fluid to drain from the base of the stem 13. This facilitates central drainage of fluid through the Gellhorn pessary 10 without compromising the structural integrity of the stem 13 that might be seen by inclusion of a central drainage port extending through the length of the stem 13.

The V-shaped supporting web 15 includes an aperture 24 and a slit 25, as illustrated in Figure 2. The aperture 24 and a slit 25 ensure no fluid build-up is possible above the V-shaped supporting web 15.

Referring to Figures 8 to 10, a Gellhorn pessary 30 is illustrated. Again, the Gellhorn pessary 30 includes a body 31 comprising a base 32 and a stem 33 extending therefrom, a finger-pull 34 associated with the body 31 that is moveable between an open orientation, as illustrated, and a retained orientation.

The finger-pull 34 again comprises a substantially V-shaped supporting web 35 hingedly connected with the base 32 of the Gellhorn pessary 30 and a loop 36 extending from the V-shaped supporting web 35.

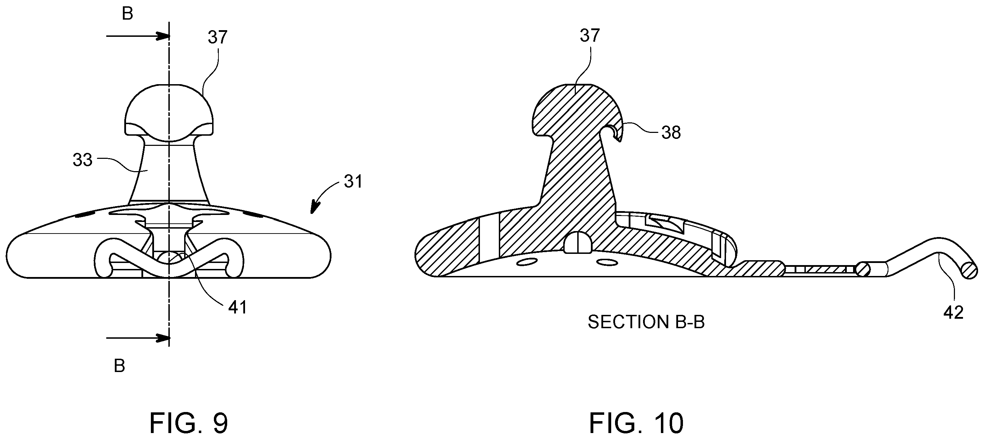

The stem 33 of the Gellhorn pessary 30 once again includes a distal knob 37 that includes a tab 38 on an underside thereof that receives a distal portion 39 of the loop 36 to retain the finger-pull 34 relative to the stem 33 in the retained orientation. To assist in the retention under the tab 38 of the distal knob 37, the distal end 39 of the loop 36 has a concave form 41,42, best seen in Figures 9 and 10, at both the distal portion 39 of the loop 36 and on the sides of the loop 36. This minimises projection of the loop 36 away from the stem 33, again potentially adding to the comfort of the user.

The base 31 again includes a recessed portion 40 on an upper surface thereof. The recessed portion 40 receives the V-shaped supporting web 35 when the finger-pull 34 is in the retained orientation. The recessed portion 40 again comprises two opposing tabs 43 extending from lateral sides of the recessed portion 80 and adapted to retain the V-shaped supporting web 75 in the recessed portion 80. The V-shaped supporting web 35 includes an aperture 44 and a slit 45.

Throughout this specification, unless the context requires otherwise, the word "comprise", or variations such as "comprises" or "comprising", will be understood to imply the inclusion of a stated step or element or integer or group of steps or elements or integers, but not the exclusion of any other step or element or integer or group of steps, elements or integers. Thus, in the context of this specification, the term "comprising" is used in an inclusive sense and thus should be understood as meaning "including principally, but not necessarily solely".

Unless the context requires otherwise or specifically stated to the contrary, integers, steps or elements of the invention recited herein as singular integers, steps or elements clearly encompass both singular and plural forms of the recited integers, steps or elements.

It will be appreciated that the foregoing description has been given by way of illustrative example of the invention and that all such modifications and variations thereto as would be apparent to persons of skill in the art are deemed to fall within the broad scope and ambit of the invention as herein set forth.

## Claims

1. A Gellhorn pessary comprising:
a body comprising a base and a stem extending therefrom; and
a finger-pull associated with the body and adapted to receive at least one finger of a user to facilitate removal of the Gellhorn pessary,
wherein said finger-pull is moveable between an open orientation that facilitates removal of the Gellhorn pessary and a retained orientation in which it is retained relative to the stem of the Gellhorn pessary.

2. The Gellhorn pessary of claim 1, wherein the finger-pull comprises a substantially V-shaped supporting web hingedly connected with the base of the Gellhorn pessary and a loop extending from the V-shaped supporting web.

3. The Gellhorn pessary of claim 2, wherein the stem of the Gellhorn pessary comprises a solid tapered shaft and a distal knob, said distal knob preferably comprising a tab on an underside thereof, said tab adapted to receive a distal portion of said loop and thereby retain said finger-pull relative to said stem in the retained orientation.

4. The Gellhorn pessary of claim 3, wherein the distal end of said loop has a concave form.

5. The Gellhorn pessary of any one of claims 2 to 4, wherein said base comprises a recessed portion on an upper surface thereof, said recessed portion adapted to receive said V-shaped supporting web when said finger-pull is in said retained orientation.

6. The Gellhorn pessary of claim 5, wherein said recessed portion comprises at least one, preferably two opposing tabs adapted to retain said V-shaped supporting web in said recessed portion, the tabs of said recessed portion preferably extending from lateral sides of said recessed portion.

7. The Gellhorn pessary of any one of claims 2 to 6, wherein said finger-pull is a unitary structure formed from a resilient polymeric material, the loop being flexible between a planar orientation relative to said V-shaped supporting web, in the open orientation, and a perpendicular orientation relative to said V-shaped supporting web, in the retained orientation.

8. The Gellhorn pessary of any one of claims 1 to 7, wherein said base comprises a plurality of drainage apertures, preferably wherein said stem comprises a central hole extending through the length of the stem.

9. The Gellhorn pessary of any one of claims 1 to 8, wherein the base of said body comprises a drainage port extending from an underside of said base, at an angle through said base of the Gellhorn pessary, to an opening preferably at a base of said stem.

10. The Gellhorn pessary of any one of claims 1 to 9, wherein said finger-pull comprises a plurality of drainage outlets, preferably, said drainage outlets formed as apertures, for example circular apertures, and/or a longitudinal slit.

11. The Gellhorn pessary of any one of claims 1 to 10, wherein the body of the Gellhorn pessary is a unitary piece of material, preferably formed from silicone.

12. The Gellhorn pessary of any one of claims 1 to 11, wherein the body and finger-pull are formed from silicone.

13. The Gellhorn pessary of any one of claims 1 to 12, wherein said Gellhorn pessary is a unitary device formed from a one-mould process.
